# EUROPEAN PATENT APPLICATION

(11) **EP 4 597 109 A1**
(43) Date of publication of application: **06.08.2025**
(21) Application number: 25150271.2
(22) Date of filing: 04.01.2025
(51) Int. Cl.: G01N 33/543, G01N 33/558, G01N 33/577, G01N 33/58, G01N 33/72

(54) **COLLOIDAL GOLD TEST PAPER FOR DOUBLE-WINDOW DETECTION OF FECAL OCCULT BLOOD AND APPLICATION THEREOF**

(30) Priority: 30.01.2024 CN 202410126271
(71) Applicant: Hangzhou Alltest Biotech Co., Ltd, Hangzhou, Zhejiang 310000 (CN)
(72) Inventor: GAO, Fei, Hangzhou, 310000 (CN); WANG, Jingjing, Hangzhou, 310000 (CN); CHEN, Jinshu, Hangzhou, 310000 (CN); ZHANG, Qiuxue, Hangzhou, 310000 (CN); LU, Weike, Hangzhou, 310000 (CN); TU, Xiaojuan, Hangzhou, 310000 (CN)
(74) Representative: JD&P Patent Attorneys Joanna Dargiewicz & Partners

(57) **Abstract**

Disclosed are colloidal gold test paper for double-window detection of fecal occult blood and an application thereof, and the colloidal gold test paper comprises a left reagent strip and a right reagent strip; the reagent strip is provided with a sample pad, a label pad, a nitrocellulose membrane and a water absorption pad; and the right reagent strip contains a mouse anti-human hemoglobin monoclonal antibody treated with a treatment solution. According to the invention, by double-window detection, the occurrence of HOOK phenomenon can be delayed, and the accuracy of colloidal gold detection can be improved.

## Description

### TECHNICAL FIELD

The present invention relates to the technical field of immunoassay, and particularly to colloidal gold test paper for double-window detection of fecal occult blood and an application thereof.

### BACKGROUND OF THE PRESENT INVENTION

Alimentary tract hemorrhage may be caused by many diseases, and is divided into revealed hemorrhage and concealed hemorrhage. An amount of physiological hemorrhage of an alimentary tract of a normal person for 24 hours is 0.6 mL. If an amount of alimentary tract hemorrhage is more than 2 mL, the alimentary tract belongs to pathologic hemorrhage. A fecal occult blood experiment is commonly used in clinic as a common screening index to detect alimentary tract hemorrhage, chronic gastrointestinal hemorrhage and early diagnosis of malignant tumors. The fecal occult blood experiment is of great significance to the early detection, early diagnosis and early treatment of diseases of alimentary tract and malignant tumors of alimentary tract, and to the improvement of survival probability and life quality of patients.

Colloidal gold detection of fecal occult blood has the advantages of rapidity, simple operation, relatively high sensitivity, the lowest detection of 100 ng/mL hemoglobin, accurate detection of asymptomatic, small-amount and persistent hemorrhage invisible to naked eyes and microscope, non-interference by drugs, iron preparations, animal blood and dietary restriction, and the like, and has strong specificity. The colloidal gold detection has gradually replaced a chemical method as a main method for detecting fecal occult blood at present.

The colloidal gold method for detecting fecal occult blood is mainly based on a principle of double-antibody sandwich method, anti-human hemoglobin polyclonal antibody and monoclonal antibody are subjected to colloidal gold labeling to specifically bind to human hemoglobin in feces, and the hemoglobin in human feces is detected by color development of a colloidal gold label. However, due to the limitation of the detection method, HOOK phenomenon is easy to occur, which leads to a false negative result of a colloidal gold result, thus affecting the correct diagnosis in clinic and delaying the best treatment opportunity. Secondly, when there are tarry black stools, bright red stools with blood or abnormal brown stools in clinic, if a test result is negative, a sample needs to be repeatedly diluted for many times and then tested again, which increases labor and time costs in clinic, and because the hemoglobin is easy to degrade, if the sample is stored at an excessively high temperature or for an excessively long period, the repeated test may still lead to an incorrect test result.

At present, most colloidal gold detection kits for fecal occult blood on the market are single-window detection kits, and when it is detected that a hemoglobin content is greater than 5 mg/mL, the result is negative or weakly positive. Therefore, the colloidal gold detection of fecal occult blood has some limitations, which is easy to have a negative impact on the accuracy and simple and fast operation of the colloidal gold detection of fecal occult blood.

### SUMMARY OF THE PRESENT INVENTION

In order to solve the above technical problems, the present invention provides colloidal gold test paper for double-window detection of fecal occult blood and an application thereof, wherein, based on an original colloidal gold test reagent strip, a detection window is added, and a mouse anti-human hemoglobin monoclonal antibody treated with a treatment solution is added in a test reagent strip in the new window, which plays a neutralizing role before a T2 test line, and can delay the occurrence of HOOK phenomenon and improve the accuracy of colloidal gold detection.

The object of the present invention is achieved by the following technical solution.

In a first aspect, the present invention provides colloidal gold test paper for double-window detection of fecal occult blood, which comprises a left reagent strip and a right reagent strip, wherein the reagent strip comprises a PVC base plate and a sample pad, a label pad, a nitrocellulose membrane and a water absorption pad which are sequentially connected and arranged on the PVC base plate; the nitrocellulose membrane starts from one side close to the label pad, a T1 test line and a C quality control line are sequentially arranged on the left reagent strip, and a T2 test line and a C quality control line are sequentially arranged on the right reagent strip; the T1 test line and the T2 test line are coated with a mouse anti-human hemoglobin monoclonal antibody, and the label pad contains a mouse anti-human hemoglobin monoclonal antibody-colloidal gold conjugate; and the right reagent strip also contains a mouse anti-human hemoglobin monoclonal antibody treated with a treatment solution, which is arranged on the sample pad or on the nitrocellulose membrane between the T2 test line and the label pad to serve as a neutralizing role before the T2 test line.

Basic principles used in the present invention are colloidal gold immunochromatography and a double-antibody sandwich method, when a sample contains hemoglobin, the hemoglobin after sample injection may bind to the mouse anti-human hemoglobin monoclonal antibody (for labeling)-colloidal gold conjugate in the label pad first to form an antigen-antibody complex, with the progress of chromatography, and the complex may be captured by the mouse anti-human hemoglobin monoclonal antibody (for coating) coated on the nitrocellulose membrane to form a mouse anti-human hemoglobin monoclonal antibody (for coating)-hemoglobin-mouse anti-human hemoglobin monoclonal antibody (for labeling)-colloidal gold conjugate, which shows a purplish red color and a clear color bar, and is visible by naked eyes without other auxiliary equipment, so that the operation is simple and convenient.

Specifically, after a high-concentration sample to be tested is added into the left reagent strip, when the sample to be tested flows through the label pad, the hemoglobin binds to the mouse anti-human hemoglobin monoclonal antibody (for labeling)-colloidal gold conjugate to form a complex, and the complex can specifically bind to the mouse anti-human hemoglobin monoclonal antibody (for coating) on the T1 test line, and however, because excessive hemoglobin in the sample may compete with the complex hemoglobin-mouse anti-human hemoglobin monoclonal antibody (for labeling)-colloidal gold conjugate to bind to the antibody on the T1 test line, the complex cannot bind to the antibody on the T1 test line, which leads to a weakly positive or negative result shown on the T1 test line.

After the high-concentration sample to be tested is added into the right reagent strip, when the sample to be tested flows through the label pad, the hemoglobin binds to the mouse anti-human hemoglobin monoclonal antibody (for labeling)-colloidal gold conjugate to form the complex hemoglobin-mouse anti-human hemoglobin monoclonal antibody (for labeling)-colloidal gold conjugate, the sample continues to move forward under an action of chromatography, excess hemoglobin binds to the mouse anti-human hemoglobin monoclonal antibody (for coating) treated with the treatment solution when the sample passes through a blocking line coated with the mouse anti-human hemoglobin monoclonal antibody (for coating) treated with the treatment solution, the sample continues to move, and then the hemoglobin-mouse anti-human hemoglobin monoclonal antibody (for labeling)-colloidal gold conjugate specifically binds to the mouse anti-human hemoglobin monoclonal antibody (for coating) on the T2 test line, so that the sample shows a purplish red band. Alternatively, after the high-concentration sample to be tested is added into the right reagent strip, when the sample to be tested flows through the sample pad, the hemoglobin binds to the mouse anti-human hemoglobin monoclonal antibody (for coating) treated with the treatment solution first, the sample continues to move forward under the action of chromatography, when the sample flows through the label pad, remaining hemoglobin binds to the mouse anti-human hemoglobin monoclonal antibody (for labeling)-colloidal gold conjugate to form the complex hemoglobin-mouse anti-human hemoglobin monoclonal antibody (for labeling)-colloidal gold conjugate, and the sample continues to move to specifically bind to the mouse anti-human hemoglobin monoclonal antibody on the T2 test line, so that the sample shows a purplish red band.

According to the present invention, whether the sample contains the hemoglobin may be judged by color development of the test line and the quality control line, a range of a hemoglobin concentration in a stool sample may also be judged by color development of two test lines, the T1 test line coated in a left reaction zone has high sensitivity and can realize the lowest detection of 100 ng/mL hemoglobin, and the T2 test line coated in a right reaction zone can realize the lowest detection of 500 ng/mL hemoglobin. By double-window detection, the occurrence of HOOK phenomenon can be delayed, and the accuracy of colloidal gold detection can be improved.

Preferably, a coating concentration of the mouse anti-human hemoglobin monoclonal antibody coated on the T1 test line is 1.0 mg/mL to 2.0 mg/mL; and a coating concentration of the mouse anti-human hemoglobin monoclonal antibody coated on the T2 test line is 1.0 mg/mL to 2.0 mg/mL.

Preferably, when the mouse anti-human hemoglobin monoclonal antibody treated with the treatment solution is on the nitrocellulose membrane between the T2 test line and the label pad, an antibody concentration is 0.5 mg/mL to 1.5 mg/mL, and when the mouse anti-human hemoglobin monoclonal antibody treated with the treatment solution is on the sample pad, the antibody concentration is 0.05 mg/mL to 0.1 mg/mL.

Specifically, when the mouse anti-human hemoglobin monoclonal antibody treated with the treatment solution is on the nitrocellulose membrane between the T2 test line and the label pad, an antibody concentration of the mouse anti-human hemoglobin monoclonal antibody treated with the treatment solution in the treatment solution is 0.5 mg/mL to 1.5 mg/mL; and when the mouse anti-human hemoglobin monoclonal antibody treated with the treatment solution is on the sample pad, an antibody concentration of the mouse anti-human hemoglobin monoclonal antibody treated with the treatment solution in a sample pad solution containing the treatment solution is 0.05 mg/mL to 0.1 mg/mL.

Preferably, the treatment solution is a mixed aqueous solution of sucrose, sodium chloride, disodium hydrogen phosphate and a chelating agent, and a pH value is adjusted to be 7.2 to 7.6.

Preferably, in the treatment solution, a content of the sucrose is 8 mg/mL to 15 mg/mL, a content of the sodium chloride is 5 g/L to 10 g/L, a content of the disodium hydrogen phosphate is 0.5 g/L to 3 g/L, and a content of the chelating agent is 5 g/L to 10 g/L; and the chelating agent is one of an amino polycarboxylic acid and a sodium salt thereof.

Preferably, the chelating agent is one of ethylenediamine tetraacetic acid (EDTA), disodium ethylenediamine tetraacetate, hydroxyethyl ethylenediamine triacetate (HEDTA), sodium hydroxyethyl ethylenediamine triacetate, nitrilotriacetic acid (NTA) and sodium aminotriacetate.

The addition of the chelating agent can improve a detection rate of the high-concentration hemoglobin, and the chelating agent can improve protein adsorption and an immune reaction speed by adjusting an ionic environment in an immune reaction system in cooperation with other ingredients in the treatment solution. Meanwhile, by adjusting concentrations of various ingredients and a pH value of the treatment solution, the ingredients may have better synergy, especially a concentration of the chelating agent has a great influence on a treatment effect of the treatment solution, and when the concentration of the chelating agent exceeds a limited range in the present invention, the detection rate of the hemoglobin will be reduced.

Preferably, the C quality control line is coated with goat anti-mouse IgG, and a coating concentration is 0.5 mg/mL to 2.0 mg/mL.

Preferably, the label pad contains the mouse anti-human hemoglobin monoclonal antibody-colloidal gold conjugate and a mouse IgG- colloidal gold conjugate; a concentration of the mouse anti-human hemoglobin monoclonal antibody-colloidal gold conjugate is OD20 to OD60; and the mouse anti-human hemoglobin monoclonal antibody (for coating) coated on the T1 test line and the T2 test line and the mouse anti-human hemoglobin monoclonal antibody (for coating) treated with the treatment solution are directed at the same epitope, while the antibody and the mouse anti-human hemoglobin monoclonal antibody (for labeling) contained on the label pad are directed at different epitopes.

The mouse anti-human hemoglobin monoclonal antibody treated with the treatment solution captures excessive hemoglobin before the test line, so as to avoid excessive hemoglobin from competing to bind to the antibody on the test line to lead to a weakly positive or false negative result, thus improving an upper limit of a detection range and delaying the HOOK phenomenon.

Preferably, the nitrocellulose membrane is arranged in the middle of the PVC base plate; two ends of the nitrocellulose membrane are respectively provided with the label pad and the water absorption pad, and are respectively connected with bottom sides of one ends of the label pad and the water absorption pad, and bottom sides of the other ends of the label pad and the water absorption pad are respectively connected with the PVC base plate; an upper side of the end of the label pad connected with the PVC base plate is provided with the sample pad; and a bottom side of one end of the sample pad is connected with the upper side of the label pad, and a bottom side of the other end of the sample pad is connected with the PVC base plate.

In a second aspect, the present invention further provides an application of the colloidal gold test paper in detecting hemoglobin, wherein a sample to be tested is added into the sample pads of the left reagent strip and the right reagent strip, and a range of a hemoglobin concentration is judged according to color development of the T1 test line and the T2 test line.

The colloidal gold test paper in the present invention may detect whether the sample contains the hemoglobin, and when the test line T and the quality control line C both show the red band, the sample contains the hemoglobin; when only the quality control line C shows the red band, the sample does not contain the hemoglobin or a hemoglobin content in the sample is lower than a detection value; and when the quality control line C does not show the red band, the test strip is invalid. The T1 test line coated in a left reaction zone has high sensitivity and can realize the lowest detection of 100 ng/mL hemoglobin, and the T2 test line coated in a right reaction zone can realize the lowest detection of 500 ng/mL hemoglobin.

The colloidal gold test paper in the present invention may detect a range of a hemoglobin concentration in the sample, the sample is added into the sample pads of the left reagent strip and the right reagent strip, and the range of the hemoglobin concentration may be judged according to color development of two test lines.

Preferably, when the T1 test line does not develop color and the T2 test line does not develop color, the hemoglobin concentration is less than 100 ng/mL; when the T1 test line develops color and the T2 test line does not develop color, the hemoglobin concentration is greater than or equal to 100 ng/mL and less than 500 ng/mL; when the T1 test line develops color and the T2 test line develops color, the hemoglobin concentration is greater than or equal to 500 ng/mL and less than 5 mg/mL; and when the T1 test line does not develop color and the T2 test line develops color, the hemoglobin concentration is greater than or equal to 5 mg/mL.

More preferably, when the T1 test line does not develop color and the T2 test line does not develop color, the hemoglobin concentration is less than 100 ng/mL; when the T1 test line develops color and the T2 test line does not develop color, the hemoglobin concentration is greater than or equal to 100 ng/mL and less than 500 ng/mL; when the T1 test line strongly develops color (stronger than the T2 test line) and the T2 test line weakly develops color (weaker than the T1 test line), the hemoglobin concentration is greater than or equal to 500 ng/mL and less than 10000 ng/mL; when the T1 test line strongly develops color and the T2 test line strongly develops color, the hemoglobin concentration is 10000 ng/mL; when T1 test line weakly develops color (weaker than the T2 test line) and the T2 test line strongly develops color (stronger than the T1 test line), the hemoglobin concentration is greater than 10000 ng/mL and less than 5 mg/mL; and when the T1 test line does not develop color and the T2 test line develops color, the hemoglobin concentration is greater than or equal to 5 mg/mL.

Compared with the prior art, the present invention has the following beneficial effects.
(1) According to the present invention, based on an original colloidal gold test reagent strip, a detection window is added, the mouse anti-human hemoglobin monoclonal antibody treated with the treatment solution is added in a test reagent strip in the new window, when the sample contains the high-concentration hemoglobin, the mouse anti-human hemoglobin monoclonal antibody treated with the treatment solution may neutralize excessive hemoglobin, and then a colloidal gold hemoglobin antigen-antibody complex specifically binds to the hemoglobin antibody on the test line, so as to show a positive result, so that double-window detection can delay the occurrence of HOOK phenomenon and improve the accuracy of colloidal gold detection.
(2) The present invention scientifically and objectively realizes rapid detection from quantitation to semi-quantitation, which not only ensures high sensitivity, but also improves an upper limit of a linear detection range and improves the accuracy of detection reagents; and moreover, the sample only needs to be treated once without repeated dilution, thus saving time and a labor cost.

### DESCRIPTION OF THE DRAWINGS

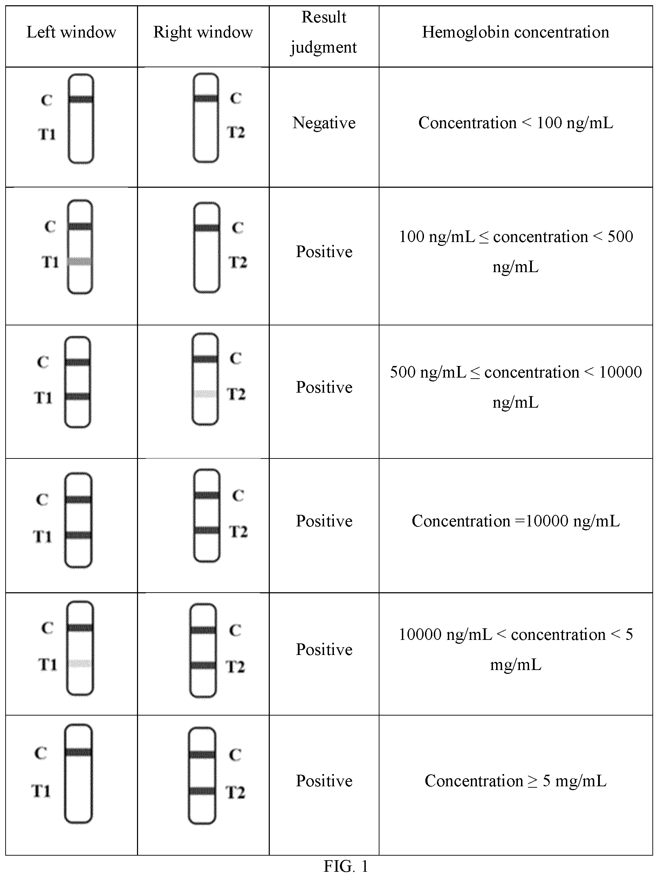

FIG. 1 is a diagram of test results of test paper according to the present invention.

### DETAILED DESCRIPTION OF PREFERRED EMBODIMENTS

The technical solution of the present invention is described hereinafter with reference to specific embodiments, but the scope of protection of the present invention is not limited to this.
(1) Preparation of colloidal gold test paper (on a right reagent strip, a mouse anti-human hemoglobin monoclonal antibody treated with a treatment solution is arranged on a nitrocellulose membrane between a T2 test line and a label pad as a blocking line)

### 1.1 Coating

1.1.1 Preparation of blocking line treatment solution: sucrose, sodium chloride, disodium hydrogen phosphate and a chelating agent are mixed and then diluted, in the treatment solution, a content of the sucrose is 8 mg/mL to 15 mg/mL, a content of the sodium chloride is 5 g/L to 10 g/L, a content of the disodium hydrogen phosphate is 0.5 g/L to 3 g/L, and a content of the chelating agent is 5 g/L to 10 g/L, and a pH value is adjusted to be 7.2 to 7.6.

1.1.2 Preparation of C/T1/T2 line coating solutions: goat anti-mouse IgG and a mouse anti-human hemoglobin monoclonal antibody (for coating) are diluted with 10 mM PBS at a pH value of 7.2 to 7.6, and coating concentrations are 0.5 mg/mL to 2.0 mg/mL, 1.0 mg/mL to 2.0 mg/mL, and 1.0 mg/mL to 2.0 mg/mL respectively.

1.1.3 Preparation of closed-line coating solution: the mouse anti-human hemoglobin monoclonal antibody (for coating) is diluted with the closed-line treatment solution, and a coating concentration is 0.5 mg/mL to 1.5 mg/mL.

1.1.4 Coating of left reagent strip: the prepared C/T1 coating solutions are respectively sprayed on a nitrocellulose membrane at a speed of 1.0 µL/cm to 1.2 µL/cm as a C quality control line and a T1 test line. A coating size of the C quality control line is 34 mm to 35 mm from a bottom edge of one end of a PVC base plate, and a coating size of the T1 test line is 28 mm to 29 mm from the bottom edge of the same end of the PVC base plate. Then, the left reagent strip is placed in a dryer at 37°C±2°C to dry for 12 hours to 24 hours, so as to obtain the coated nitrocellulose membrane.

1.1.5 Coating of right reagent strip: the prepared C/T2/blocking line coating solutions are respectively sprayed on a nitrocellulose membrane at a speed of 1.0 µL/cm to 1.2 µL/cm as a C quality control line, a T2 test line and a blocking line. A coating size of the C quality control line is 34 mm to 35 mm from a bottom edge of one end of a PVC base plate; a coating size of the T2 test line is 28 mm to 29 mm from the bottom edge of the same end of the PVC base plate; and a coating size of the closed wire is 22 mm to 23 mm from the bottom edge of the same end of the PVC base plate. Then, the right reagent strip is placed in an electric heating air-blowing dryer at 37°C±2°C to dry for 12 hours to 24 hours, so as to obtain the coated nitrocellulose membrane.

### 1.2 Labeling

1.2.1 Preparation of colloidal gold: colloidal gold particles are mixed with the mouse anti-human hemoglobin monoclonal antibody (for labeling) and mouse IgG respectively, and labeled into mouse an anti-human hemoglobin monoclonal antibody (for labeling)-colloidal gold conjugate and a mouse IgG- colloidal gold conjugate.

1.2.2 Preparation of labeling solution: the mouse anti-human hemoglobin monoclonal antibody (for labeling)-colloidal gold conjugate and the mouse IgG- colloidal gold conjugate are diluted into labeling solutions at OD20 to OD60 and OD10 to OD60 with a diluent containing 50 g/L to 55 g/L sucrose, 8 g/L to 15 g/L BSA and 6 g/L to 9 g/L disodium hydrogen phosphate.

1.2.3 The prepared labeling solution is treated onto a polyester cellulose membrane at a speed of 1.8 µL/cm to 2.0 µL/cm, and then placed in an electric heating air-blowing dryer at 37°C±2°C to dry for 12 hours to 24 hours, so as to obtain the label pad.

### 1.3 Sample pad

1.3.1 Preparation of sample pad solution: an aqueous solution containing 3 g/L to 6 g/L casein, 4 g/L to 7 g/L trihydroxymethyl aminomethane, 0.1 mL/L to 0.4 mL/L Proclin300, 3 g/L to 8 g/L PVP-10 and 0.2 mL/L to 0.7 mL/L Tween 20 is prepared, and a pH value is 7.2 to 7.6.

1.3.2 Preparation of sample pad: the sample pad solution is spread on a glass fiber, and placed in an electric heating air-blowing dryer at 37°C±2°C to dry for 12 hours to 24 hours, so as to obtain the sample pad.

### 1.4 Assembly

An assembling method of the reagent strip above is that: the nitrocellulose membrane is arranged in the middle of the PVC base plate; two ends of the nitrocellulose membrane are respectively provided with the label pad and the water absorption pad, and are respectively connected with bottom sides of one ends of the label pad and the water absorption pad, and bottom sides of the other ends of the label pad and the water absorption pad are respectively connected with the PVC base plate; an upper side of the end of the label pad connected with the PVC base plate is provided with the sample pad; and a bottom side of one end of the sample pad is connected with the upper side of the label pad, and a bottom side of the other end of the sample pad is connected with the PVC base plate. The left reagent strip and the right reagent strip are cut into a required width as required, and assembled in parallel to obtain the colloidal gold test paper for double-window detection.

(2) Preparation of colloidal gold test paper (on a right reagent strip, a mouse anti-human hemoglobin monoclonal antibody treated with a treatment solution is arranged on a sample pad)

### 1.1 Coating

1.1.1 Preparation of C/T1/T2 line coating solutions: it is the same as 1.1.2 in (1).

1.1.2 Coating of left reagent strip: it is the same as 1.1.4 in (1).

1.1.3 Coating of right reagent strip: the prepared C/T2 coating solutions are respectively sprayed on a nitrocellulose membrane at a speed of 1.0 µL/cm to 1.2 µL/cm as a C quality control line and a T2 test line. A coating size of the C quality control line is 34 mm to 35 mm from a bottom edge of one end of a PVC base plate; and a coating size of the T2 test line is 28 mm to 29 mm from the bottom edge of the same end of the PVC base plate. Then, the right reagent strip is placed in an electric heating air-blowing dryer at 37°C±2°C to dry for 12 hours to 24 hours, so as to obtain the coated nitrocellulose membrane.

### 1.2 Labeling

1.2.1 Preparation of colloidal gold: it is the same as 1.2.1 in (1).

1.2.2 Preparation of label solution: it is the same as 1.2.2 in (1).

1.2.3 Acquisition of label pad: it is the same as 1.2.3 in (1).

### 1.3 Sample pad

1.3.1 Preparation of sample pad solution: an aqueous solution containing 3 g/L to 6 g/L casein, 4 g/L to 7 g/L trihydroxymethyl aminomethane, 0.1 mL/L to 0.4 mL/L Proclin300, 3 g/L to 8 g/L PVP-10, 0.2 mL/L to 0.7 mL/L Tween 20, 8 mg/mL to 15 mg/mL sucrose, 5 g/L to 10 g/L sodium chloride, 0.5 g/L to 3 g/L disodium hydrogen phosphate, 5 g/L to 10 g/L chelating agent and 0.05 mg/mL to 0.1 mg/mL mouse anti-human hemoglobin monoclonal antibody (for coating) is prepared, and a pH value is 7.2 to 7.6.

1.3.2 Preparation of sample pad: it is the same as 1.3.2 in (1).

1.4 Assembly: it is the same as 1.4 in (1).

### Embodiment 1: Preparation of colloidal gold test paper (with right reagent strip containing blocking line)

### 1.1 Coating

1.1.1 Preparation of blocking line treatment solution: 80 mL of purified water was weighed first, then 1 g of sucrose, 0.8 g of sodium chloride, 0.2 g of disodium hydrogen phosphate and 1 g of ethylenediamine tetraacetic acid were sequentially added and stirred to be completely dissolved, then a pH value was adjusted to be 7.4, and the purified water was added to a constant volume of 100 mL.

1.1.2 Preparation of C/T1/T2 line coating solutions: goat anti-mouse IgG and a mouse anti-human hemoglobin monoclonal antibody (for coating) were diluted with 10 mM PBS at a pH value of 7.4 to prepare into 1.0 mg/mL, 1.5 mg/mL, and 1.5 mg/mL solutions respectively.

1.1.3 Preparation of closed-line coating solution: the mouse anti-human hemoglobin monoclonal antibody (for coating) was diluted with the closed-line treatment solution to prepare into 0.5 mg/mL solution.

1.1.4 Coating of left reagent strip: the prepared C/T1 coating solutions were respectively sprayed on a nitrocellulose membrane at a speed of 1.0 µL/cm as a C quality control line and a T1 test line. A coating size of the C quality control line was 34.5 mm from a bottom edge of one end of a PVC base plate, and a coating size of the T1 test line was 28.5 mm from the bottom edge of the same end of the PVC base plate. Then, the left reagent strip was placed in a dryer at 37°C±2°C to dry for 16 hours, so as to obtain the coated nitrocellulose membrane.

1.1.5 Coating of right reagent strip: the prepared C/T2/blocking line coating solutions were respectively sprayed on a nitrocellulose membrane at a speed of 1.0 µL/cm as a C quality control line, a T2 test line and a blocking line. A coating size of the C quality control line was 34.5 mm from a bottom edge of one end of a PVC base plate; a coating size of the T2 test line was 28.5 mm from the bottom edge of the same end of the PVC base plate; and a coating size of the closed wire was 22.5 mm from the bottom edge of the same end of the PVC base plate. Then, the right reagent strip was placed in an electric heating air-blowing dryer at 37°C±2°C to dry for 16 hours, so as to obtain the coated nitrocellulose membrane.

### 1.2 Labeling

1.2.1 Preparation of colloidal gold: colloidal gold particles were mixed with the mouse anti-human hemoglobin monoclonal antibody (for labeling) and mouse IgG in proportion respectively, and labeled into mouse an anti-human hemoglobin monoclonal antibody (for labeling)-colloidal gold conjugate and a mouse IgG- colloidal gold conjugate, and absorbances were measured to be OD129.5 and OD130.1 respectively by an ultraviolet spectrophotometer.

1.2.2 Preparation of labeling solution: the mouse anti-human hemoglobin monoclonal antibody (for labeling)-colloidal gold conjugate and the mouse IgG- colloidal gold conjugate were diluted into labeling solutions at OD20 and OD10 with a diluent containing 50 g/L sucrose, 10 g/L BSA and 7 g/L disodium hydrogen phosphate.

1.2.3 The prepared labeling solution was treated onto a polyester cellulose membrane at a speed of 2.0 µL/cm, and then placed in an electric heating air-blowing dryer at 37°C±2°C to dry for 16 hours, so as to obtain the label pad.

### 1.3 Sample pad

1.3.1 Preparation of sample pad solution: 80 mL of purified water was weighed first, then 0.4 g of casein, 0.48 g of trihydroxymethyl aminomethane, 0.016 mL of Proclin300, 0.5 g of PVP-10 and 0.05 mL of Tween 20 were sequentially added to be completely dissolved, then a pH value was adjusted to be 7.4, and finally, the purified water was added to a constant volume of 100 mL.

1.3.2 Preparation of sample pad: 3.5 mL of sample pad solution was spread on 17 mm*31 mm glass fiber, and placed in an electric heating air-blowing dryer at 37°C±2°C to dry for 16 hours, so as to obtain the sample pad.

### 1.4 Assembly

The water adsorption pad, the sample pad, the label pad, the coated nitrocellulose membrane and the PVC base plate were sequentially connected and assembled, and cut into a required width as required, so as to obtain the colloidal gold test paper for double-window detection.

### Embodiment 2: Preparation of colloidal gold test paper (with right reagent strip containing blocking line)

Except for the step 1.1.3, this embodiment was the same as Embodiment 1.

1.1.3 Preparation of blocking line coating solution: a preparation concentration was 1 mg/mL.

### Embodiment 3: Preparation of colloidal gold test paper (with right reagent strip containing blocking line)

Except for the step 1.1.3, this embodiment was the same as Embodiment 1.

1.1.3 Preparation of blocking line coating solution: a preparation concentration was 1.5 mg/mL.

### Embodiment 4: Preparation of colloidal gold test paper (with right reagent strip containing blocking line)

Except for the step 1.1.1 (the chelating agent), this embodiment was the same as Embodiment 1.

1.1.1 Preparation of blocking line treatment solution: 80 mL of purified water was weighed first, then 1 g of sucrose, 0.8 g of sodium chloride, 0.2 g of disodium hydrogen phosphate and 1 g of hydroxyethyl ethylenediamine triacetic acid were sequentially added and stirred to be completely dissolved, then a pH value was adjusted to be 7.4, and the purified water was added to a constant volume of 100 mL.

### Embodiment 5: Preparation of colloidal gold test paper (with right reagent strip containing blocking line)

Except for the step 1.1.1 (the chelating agent), this embodiment was the same as Embodiment 1.

1.1.1 Preparation of blocking line treatment solution: 80 mL of purified water was weighed first, then 1 g of sucrose, 0.8 g of sodium chloride, 0.2 g of disodium hydrogen phosphate and 1 g of aminotriacetic acid were sequentially added and stirred to be completely dissolved, then a pH value was adjusted to be 7.4, and the purified water was added to a constant volume of 100 mL.

### Embodiment 6: Preparation of colloidal gold test paper (with right reagent strip containing blocking line)

Except for the step 1.1.1 (the addition amount of the chelating agent), this embodiment was the same as Embodiment 1.

1.1.1 Preparation of blocking line treatment solution: 80 mL of purified water was weighed first, then 1 g of sucrose, 0.8 g of sodium chloride, 0.2 g of disodium hydrogen phosphate and 0.8 g ethylenediamine tetraacetic acid were sequentially added and stirred to be completely dissolved, then a pH value was adjusted to be 7.4, and the purified water was added to a constant volume of 100 mL.

### Embodiment 7: On a right reagent strip, a mouse anti-human hemoglobin monoclonal antibody treated with a treatment solution was arranged on a sample pad to prepare colloidal gold test paper (with the right reagent strip not containing a blocking line)

### 1.1 Coating

1.1.1 Preparation of C/T1/T2 line coating solutions: goat anti-mouse IgG and a mouse anti-human hemoglobin monoclonal antibody (for coating) were diluted with 10 mM PBS at a pH value of 7.4 to prepare into 1.0 mg/mL, 1.5 mg/mL, and 1.5 mg/mL solutions respectively.

1.1.2 Coating of left reagent strip: the prepared C/T1 coating solutions were respectively sprayed on a nitrocellulose membrane at a speed of 1.0 µL/cm as a C quality control line and a T1 test line. A coating size of the C quality control line was 34.5 mm from a bottom edge of one end of a PVC base plate, and a coating size of the T1 test line was 28.5 mm from the bottom edge of the same end of the PVC base plate. Then, the left reagent strip was placed in a dryer at 37°C±2°C to dry for 12 hours to 24 hours, so as to obtain the coated nitrocellulose membrane.

1.1.3 Coating of right reagent strip: the prepared C/T2 coating solutions were respectively sprayed on a nitrocellulose membrane at a speed of 1.0 µL/cm as a C quality control line and a T2 test line. A coating size of the C quality control line was 34.5 mm from a bottom edge of one end of a PVC base plate; and a coating size of the T2 test line was 28.5 mm from the bottom edge of the same end of the PVC base plate. Then, the right reagent strip was placed in an electric heating air-blowing dryer at 37°C±2°C to dry for 16 hours, so as to obtain the coated nitrocellulose membrane.

### 1.2 Labeling

1.2.1 Preparation of colloidal gold: colloidal gold particles were mixed with the mouse anti-human hemoglobin monoclonal antibody (for labeling) and mouse IgG in proportion respectively, and labeled into mouse an anti-human hemoglobin monoclonal antibody (for labeling)-colloidal gold conjugate and a mouse IgG- colloidal gold conjugate, and absorbances were measured to be OD129.5 and OD130.1 respectively by an ultraviolet spectrophotometer.

1.2.2 Preparation of labeling solution: the mouse anti-human hemoglobin monoclonal antibody (for labeling)-colloidal gold conjugate and the mouse IgG- colloidal gold conjugate were diluted into labeling solutions at OD20 and OD10 with a diluent containing 50 g/L sucrose, 10 g/L BSA and 7 g/L disodium hydrogen phosphate.

1.2.3 The prepared labeling solution was treated onto a polyester cellulose membrane at a speed of 2.0 µL/cm, and then placed in an electric heating air-blowing dryer at 37°C±2°C to dry for 16 hours, so as to obtain the label pad.

### 1.3 Sample pad

1.3.1 Preparation of sample pad solution: 80 mL of purified water was weighed first, then 0.4 g of casein, 0.48 g of trihydroxymethyl aminomethane, 0.016 mL of Proclin300, 0.5 g of PVP-10, 0.05 mL of Tween 20, 1 g of sucrose, 0.8 g of sodium chloride, 0.2 g of disodium hydrogen phosphate, 1 g of ethylenediamine tetraacetic acid, and 1.02 mL of mouse anti-human hemoglobin antibody (for coating) (an original concentration was 4.9 mg/mL, and a concentration in the sample pad solution was 0.05 mg/mL) were sequentially added and stirred to be completely dissolved, then a pH value was adjusted to be 7.4, and the purified water was added to a constant volume of 100 mL.

1.3.2 Preparation of sample pad: 3.5 mL of the solution was spread on 17 mm*31 mm glass fiber, and placed in an electric heating air-blowing dryer at 37°C±2°C to dry for 16 hours, so as to obtain the sample pad.

### 1.4 Assembly

The water adsorption pad, the sample pad, the label pad, the coated nitrocellulose membrane and the PVC base plate were sequentially connected and assembled, and cut into a required width as required, so as to obtain the colloidal gold test paper for double-window detection.

### Comparative Example 1: Preparation of colloidal gold test paper (with right reagent strip containing blocking line)

Except for the step 1.1.3, this embodiment was the same as Embodiment 1.

1.1.3 Preparation of blocking line coating solution: a preparation concentration was 0.1 mg/mL.

### Comparative Example 2: Preparation of colloidal gold test paper (with right reagent strip containing blocking line)

Except for the step 1.1.3, this embodiment was the same as Embodiment 1.

1.1.3 Preparation of blocking line coating solution: a preparation concentration was 2.0 mg/mL.

### Comparative Example 3: Preparation of colloidal gold test paper (with right reagent strip containing blocking line)

Except for the step 1.1.1 (no addition of the chelating agent), this embodiment was the same as Embodiment 1.

1.1.1 Preparation of blocking line treatment solution: 80 mL of purified water was weighed first, then 1 g of sucrose, 0.8 g of sodium chloride and 0.2 g of disodium hydrogen phosphate were sequentially added and stirred to be completely dissolved, then a pH value was adjusted to be 7.4, and the purified water was added to a constant volume of 100 mL.

### Comparative Example 4: Preparation of colloidal gold test paper (with right reagent strip containing blocking line)

Except for the step 1.1.1 (excessively high addition amount of the chelating agent), this embodiment was the same as Embodiment 1.

1.1.1 Preparation of blocking line treatment solution: 80 mL of purified water was weighed first, then 1 g of sucrose, 0.8 g of sodium chloride, 0.2 g of disodium hydrogen phosphate and 1.2 g ethylenediamine tetraacetic acid were sequentially added and stirred to be completely dissolved, then a pH value was adjusted to be 7.4, and the purified water was added to a constant volume of 100 mL.

### Comparative Example 5: Preparation of colloidal gold test paper (with right reagent strip containing blocking line)

Except for the step 1.1.1 and the step 1.1.3, this embodiment was the same as Embodiment 1, which was specifically as follows.

The step 1.1.1 was removed, and in the step 1.1.3 of preparation of closed-line coating solution: the mouse anti-human hemoglobin monoclonal antibody (for coating) was diluted with 10 mM PBS buffer at a pH value of 7.4 (which was the same as the solution prepared for coating the C/T1/T2 lines) to prepare into 0.5 mg/mL solution.

The embodiments and the comparative examples were subjected to a performance test, which was carried out with a negative quality control, 50 ng/mL hemoglobin, 100 ng/mL hemoglobin, 200 ng/mL hemoglobin, 500 ng/mL hemoglobin, 1 µg/mL hemoglobin, 10 µg/mL hemoglobin, 100 µg/mL hemoglobin, 5 mg/mL hemoglobin, 10mg/mL hemoglobin and 50 mg/mL hemoglobin respectively, and the test of each concentration was set to be repeated for ten times. Test results were shown in Table 1 and Table 2, and results of strong and weak color development were shown in FIG. 1.

**Table 1**

| Hemoglobin concentration | Embodiment 1 | | Embodiment 2 | | Embodiment 3 | | Embodiment 4 | | Embodiment 5 | | Embodiment 6 | | Embodiment 7 | |
|---|---|---|---|---|---|---|---|---|---|---|---|---|---|---|
| | Left window | Right window | Left window | Right window | Left window | Right window | Left window | Right window | Left window | Right window | Left window | Right window | Left window | Right window |
| Negative quality control product | 10- | 10- | 10- | 10- | 10- | 10- | 10- | 10- | 10- | 10- | 10- | 10- | 10- | 10- |
| 50 ng/mL | 10- | 10- | 10- | 10- | 10- | 10- | 10- | 10- | 10- | 10- | 10- | 10- | 10- | 10- |
| 100 ng/mL | 10+ | 10- | 10+ | 10- | 10+ | 10- | 10+ | 10- | 10+ | 10- | 10+ | 10- | 10+ | 10- |
| 200 ng/mL | 10+ | 10- | 10+ | 10- | 10+ | 10- | 10+ | 10- | 10+ | 10- | 10+ | 10- | 10+ | 10- |
| 500 ng/mL | 10+ strong | 10+ weak | 10+ strong | 10+ weak | 10+ strong | 10+ weak | 10+ strong | 10+ weak | 10+ strong | 10+ weak | 10+ strong | 10+ weak | 10+ strong | 10+ weak |
| 1 µg/mL | 10+ strong | 10+ weak | 10+ strong | 10+ weak | 10+ strong | 10+ weak | 10+ strong | 10+ weak | 10+ strong | 10+ weak | 10+ strong | 10+ weak | 10+ strong | 10+ weak |
| 10 µg/mL | 10+ strong | 10+ strong | 10+ strong | 10+ strong | 10+ strong | 10+ strong | 10+ strong | 10+ strong | 10+ strong | 10+ strong | 10+ strong | 10+ strong | 10+ strong | 10+ strong |
| 100 µg/mL | 10+ weak | 10+ strong | 10+ weak | 10+ strong | 10+ weak | 10+ strong | 10+ weak | 10+ strong | 10+ weak | 10+ strong | 10+ weak | 10+ strong | 10+ weak | 10+ strong |
| 5 mg/mL | 10- | 10+ | 10- | 10+ | 10- | 10+ | 10- | 10+ | 10- | 10+ | 10- | 10+ | 10- | 10+ |
| 10 mg/mL | 10- | 10+ | 10- | 10+ | 10- | 10+ | 10- | 10+ | 10- | 10+ | 10- | 10+ | 10- | 10+ |
| 50 mg/mL | 10- | 10+ | 10- | 10+ | 10- | 10+ | 10- | 10+ | 10- | 10+ | 10- | 10+ | 10- | 10+ |

| | | | | | | | | | | | | | | |
|---|---|---|---|---|---|---|---|---|---|---|---|---|---|---|
| * Note: "+" indicates that the test result is positive; "-" indicates that the test result is negative; 10(+) indicates that the 10 test results are all positive; and 10(-) indicates that the 10 test results are all negative. | | | | | | | | | | | | | | |

**Table 2**

| Hemoglobin concentration | Comparative Example 1 | | Comparative Example 2 | | Comparative Example 3 | | Comparative Example 4 | | Comparative Example 5 | |
|---|---|---|---|---|---|---|---|---|---|---|
| | Left window | Right window | Left window | Right window | Left window | Right window | Left window | Right window | Left window | Right window |
| Negative quality control product | 10- | 10- | 10- | 10- | 10- | 10- | 10- | 10- | 10- | 10- |
| 50 ng/mL | 10- | 10- | 10- | 10- | 10- | 10- | 10- | 10- | 10- | 10- |
| 100 ng/mL | 10+ | 10- | 10+ | 10- | 10+ | 10- | 10+ | 10- | 10+ | 10- |
| 200 ng/mL | 10+ Strong | 10+ weak | 10+ | 10- | 10+ | 10- | 10+ | 10- | 10+ | 10- |
| 500 ng/mL | 10+ strong | 10+ weak | 10+ | 10- | 10+ strong | 10+ weak | 10+ strong | 10+ weak | 10+ strong | 10+ weak |
| 1 µg/mL | 10+ strong | 10+ strong | 10+ | 10- | 10+ strong | 10+ weak | 10+ strong | 10+ weak | 10+ strong | 10+ weak |
| 10 µg/mL | 10+ strong | 10+ weak | 10+ | 10- | 10+ strong | 10+ strong | 10+ strong | 10+ weak | 10+ strong | 10+ weak |
| 100 µg/mL | 10+ weak | 10+ weak | 10+ weak | 10+ weak | 10+ weak | 10+ strong | 10+ weak | 10+ weak | 10+ weak | 10+ weak |
| 5 mg/mL | 10- | 10+ | 10- | 10+ | 10- | 10+ | 10- | 10- | 10- | 10- |
| 10 mg/mL | 10- | 10- | 10- | 10+ | 10- | 10+ | 10- | 10- | 10- | 10- |
| 50 mg/mL | 10- | 10- | 10- | 10+ | 10- | 6+/4- | 10- | 10- | 10- | 10- |

| | | | | | | | | | | |
|---|---|---|---|---|---|---|---|---|---|---|
| * Note: "+" indicates that the test result is positive; "-" indicates that the test result is negative; 10(+) indicates that the 10 test results are all positive; and 10(-) indicates that the 10 test results are all negative. | | | | | | | | | | |

Result analysis: as shown in Table 1 and Table 2, in an immunochromatographic reaction, Comparative Example 1 and Comparative Example 2 show that, if a composition or a use amount of the blocking line is inappropriate, the reaction environment of an antigen and an antibody will be affected, the binding of the antigen and the antibody is affected, and finally, the accuracy of a detection result is affected. Within a range of concentration limited in the present invention, the mouse anti-human hemoglobin monoclonal antibody treated with the treatment solution in the right reagent strip can delay the occurrence of HOOK phenomenon and improve the accuracy of colloidal gold detection, especially for a high-concentration sample, a positive result can be accurately obtained without repeated dilution and retest, and meanwhile, ranges of hemoglobin concentration and content may be judged according to results of double windows.

Meanwhile, by treating the blocking line treatment solution, it can be seen from the results of Embodiment 1 and Comparative Example 3 that the addition of the chelating agent in the blocking line treatment solution can improve a detection rate of the high-concentration hemoglobin and the chelating agent can improve protein adsorption and an immune reaction speed by adjusting an ionic environment in an immune reaction system. However, Comparative Example 4 shows that the chelating agent with an excessively high concentration will affect the binding of the hemoglobin to the mouse anti-human monoclonal antibody, so that excess hemoglobin cannot be neutralized, and the HOOK effect cannot be delayed. Meanwhile, Comparative Example 5 shows that, if the blocking line treatment solution has conventional PBS ingredients, there may still be a negative result in detection of a high-concentration hemoglobin sample.

### Verification by clinical sample

According to the present invention, a total of 26 positive samples of fecal occult blood were collected, measurement values were confirmed by a third-party measuring reagent (Shanghai Toujing Diagnostic Technology Co., Ltd.-Hemoglobin/Transferrin Determination Kit (Flow Fluorescence Method)), and test results were shown in Table 3. There were 11 positive samples with a hemoglobin concentration of 100 ng/mL to 500 ng/mL, 9 positive samples with a hemoglobin concentration of 500 ng/mL to 5 mg/mL, and 6 high-concentration positive samples with a hemoglobin concentration greater than 5 mg/mL.

**Table 3**

| Name of sample | Hemoglobin concentration | Embodiment 1 | | Comparative Example 3 | | Comparative Example 4 | |
|---|---|---|---|---|---|---|---|
| | | Left window | Right window | Left window | Right window | Left window | Right window |
| Sample 1 | 236.9 ng/mL | + | - | + | - | + | - |
| Sample 2 | 503.6 µg/mL | +weak | +strong | +weak | +weak | +weak | +weak |
| Sample 3 | 1046.5 µg/mL | +weak | +strong | +weak | +weak | +weak | +weak |
| Sample 4 | 109.6 ng/mL | + | - | + | - | + | - |
| Sample 5 | 185.4 ng/mL | + | - | + | - | + | - |
| Sample 6 | 763.9 µg/mL | +weak | +strong | +weak | +weak | +weak | +weak |
| Sample 7 | 516.9 ng/mL | +strong | +weak | +strong | +weak | +strong | +weak |
| Sample 8 | 2538.6 µg/mL | +weak | +strong | +weak | +weak | +weak | +weak |
| Sample 9 | 17.9 mg/mL | - | + | - | - | - | - |
| Sample 10 | 264.1 ng/mL | + | - | + | - | + | - |
| Sample 11 | 507.2 ng/mL | +strong | +weak | +strong | +weak | +strong | +weak |
| Sample 12 | 13.4 mg/mL | - | + | - | - | - | - |
| Sample 13 | 336.7 ng/mL | + | - | + | - | + | - |
| Sample 14 | 452.6 ng/mL | + | - | + | - | + | - |
| Sample 15 | 56.2 mg/mL | - | + | - | - | - | - |
| Sample 16 | 127.9 ng/mL | + | - | + | - | + | - |
| Sample 17 | 71.3 mg/mL | - | + | - | - | - | - |
| Sample 18 | 379.8 ng/mL | + | - | + | - | + | - |
| Sample 19 | 285.6 ng/mL | + | - | + | - | + | - |
| Sample 20 | 481.4 ng/mL | + | - | + | - | + | - |
| Sample 21 | 20.9 mg/mL | - | + | - | - | - | - |
| Sample 22 | 4913.5 µg/mL | +weak | +strong | +weak | +weak | +weak | +weak |
| Sample 23 | 569.4 ng/mL | +strong | +weak | +strong | +weak | +strong | +weak |
| Sample 24 | 32.7 mg/mL | - | + | - | - | - | - |
| Sample 25 | 289.3 µg/mL | +weak | +strong | +weak | +weak | +weak | +weak |
| Sample 26 | 429.8 ng/mL | + | - | + | - | + | - |

| | | | | | | | |
|---|---|---|---|---|---|---|---|
| * Note: "+" indicates that the test result is positive; and "-" indicates that the test result is negative. | | | | | | | |

As shown in Table 3, it can be seen from the test results of the clinical samples that Embodiment 1 has high sensitivity and accuracy, especially for the high-concentration hemoglobin sample, the range of the hemoglobin concentration in the sample can be judged according to color development results of two T lines at the same time. However, for Comparative Example 3 and Comparative Example 4, the absence or excessive addition of the chelating agent in the treatment solution will both affect the detection accuracy of the colloidal gold test paper.

The above are only the preferred embodiments of the present invention, and do not limit the patent scope of the present invention. Any equivalent structure or equivalent process transformations made by using the specification of the present invention, or directly or indirectly applied in other related technical fields are equally included in the scope of protection of the patent of the present invention.

## Claims

1. Colloidal gold test paper for double-window detection of fecal occult blood, comprising a left reagent strip and a right reagent strip, **characterized in that** the reagent strip comprises a PVC base plate and a sample pad, a label pad, a nitrocellulose membrane and a water absorption pad which are sequentially connected and arranged on the PVC base plate; the nitrocellulose membrane starts from one side close to the label pad, a T1 test line and a C quality control line are sequentially arranged on the left reagent strip, and a T2 test line and a C quality control line are sequentially arranged on the right reagent strip; the T1 test line and the T2 test line are coated with a mouse anti-human hemoglobin monoclonal antibody, and the label pad contains a mouse anti-human hemoglobin monoclonal antibody-colloidal gold conjugate; and the right reagent strip also contains a mouse anti-human hemoglobin monoclonal antibody treated with a treatment solution, which is arranged on the sample pad or on the nitrocellulose membrane between the T2 test line and the label pad.

2. The colloidal gold test paper for double-window detection of fecal occult blood according to claim 1, **characterized in that** a coating concentration of the mouse anti-human hemoglobin monoclonal antibody coated on the T1 test line is 1.0 mg/mL to 2.0 mg/mL; and a coating concentration of the mouse anti-human hemoglobin monoclonal antibody coated on the T2 test line is 1.0 mg/mL to 2.0 mg/mL.

3. The colloidal gold test paper for double-window detection of fecal occult blood according to claim 1, **characterized in that**, when the mouse anti-human hemoglobin monoclonal antibody treated with the treatment solution is on the nitrocellulose membrane between the T2 test line and the label pad, an antibody concentration is 0.5 mg/mL to 1.5 mg/mL, and when the mouse anti-human hemoglobin monoclonal antibody treated with the treatment solution is on the sample pad, the antibody concentration is 0.05 mg/mL to 0.1 mg/mL.

4. The colloidal gold test paper for double-window detection of fecal occult blood according to claim 1, **characterized in that** the treatment solution is a mixed aqueous solution of sucrose, sodium chloride, disodium hydrogen phosphate and a chelating agent.

5. The colloidal gold test paper for double-window detection of fecal occult blood according to claim 4, **characterized in that**, in the treatment solution, a content of the sucrose is 8 mg/mL to 15 mg/mL, a content of the sodium chloride is 5 g/L to 10 g/L, a content of the disodium hydrogen phosphate is 0.5 g/L to 3 g/L, and a content of the chelating agent is 5 g/L to 10 g/L; and the chelating agent is one of an amino polycarboxylic acid and a sodium salt thereof.

6. The colloidal gold test paper for double-window detection of fecal occult blood according to claim 1, **characterized in that** the C quality control line is coated with goat anti-mouse IgG, and a coating concentration is 0.5 mg/mL to 2.0 mg/mL.

7. The colloidal gold test paper for double-window detection of fecal occult blood according to any one of claims 1 to 6, **characterized in that** the label pad contains the mouse anti-human hemoglobin monoclonal antibody-colloidal gold conjugate and a mouse IgG-colloidal gold conjugate; a concentration of the mouse anti-human hemoglobin monoclonal antibody-colloidal gold conjugate is OD20 to OD60; and the antibody in the mouse anti-human hemoglobin monoclonal antibody-colloidal gold conjugate is the mouse anti-human hemoglobin monoclonal antibody different from the antibody treated with the treatment solution on the right reagent strip and the antibody coated on the T1 test line and the T2 test line.

8. The colloidal gold test paper for double-window detection of fecal occult blood according to any one of claims 1 to 6, **characterized in that** the nitrocellulose membrane is arranged in the middle of the PVC base plate; two ends of the nitrocellulose membrane are respectively provided with the label pad and the water absorption pad, and are respectively connected with bottom sides of one ends of the label pad and the water absorption pad, and bottom sides of the other ends of the label pad and the water absorption pad are respectively connected with the PVC base plate; an upper side of the end of the label pad connected with the PVC base plate is provided with the sample pad; and a bottom side of one end of the sample pad is connected with the upper side of the label pad, and a bottom side of the other end of the sample pad is connected with the PVC base plate.

9. An application of the colloidal gold test paper for double-window detection of fecal occult blood according to any one of claims 1 to 8 in detecting hemoglobin, **characterized in that** a sample to be tested is added into the sample pads of the left reagent strip and the right reagent strip, and a range of a hemoglobin concentration is judged according to color development of the T1 test line and the T2 test line.

10. The application according to claim 9, **characterized in that**, when the T1 test line does not develop color and the T2 test line does not develop color, the hemoglobin concentration is less than 100 ng/mL; when the T1 test line develops color and the T2 test line does not develop color, the hemoglobin concentration is greater than or equal to 100 ng/mL and less than 500 ng/mL; when the T1 test line develops color and the T2 test line develops color, the hemoglobin concentration is greater than or equal to 500 ng/mL and less than 5 mg/mL; and when the T1 test line does not develop color and the T2 test line develops color, the hemoglobin concentration is greater than or equal to 5 mg/mL.
